# EUROPEAN PATENT APPLICATION

(11) **EP 4 226 919 A1**
(43) Date of publication of application: **16.08.2023**
(21) Application number: 21877743.1
(22) Date of filing: 08.10.2021
(51) Int. Cl.: A61K 31/05, A61K 31/122, A61K 31/336, A61P 1/16, A61P 3/00, A61P 3/02, A61P 39/06, A61P 43/00

(54) **FERROPTOSIS INHIBITOR**

(30) Priority: 09.10.2020 JP 2020171220
(71) Applicant: Tohoku University, Sendai-shi, Miyagi 980-8577 (JP)
(72) Inventor: MISHIMA, Eikan, Sendai-shi, Miyagi 980-8577 (JP); NAKAGAWA, Kiyotaka, Sendai-shi, Miyagi 980-8577 (JP); ITO, Junya, Sendai-shi, Miyagi 980-8577 (JP); YAMADA, Kenichi, Fukuoka-shi, Fukuoka 819-0395 (JP)
(74) Representative: MERH-IP Matias Erny Reichl Hoffmann Patentanwälte PartG mbB
(86) International application number: PCT/JP2021/037317
(87) International publication number: WO 2022/075444

(57) **Abstract**

An object of the present invention is to provide a low molecular weight compound having an anti-ferroptosis activity and an ability to scavenge lipid peroxy radicals which is relatively highly safe when ingested, can be orally ingested, and can be manufactured at a relatively low cost. The present invention provides a ferroptosis inhibitor, an agent for preventing or treating a disease associated with ferroptosis, a lipid peroxy radical scavenger, or an agent for preventing or treating a disease caused by lipid peroxide accumulation and/or lipid peroxy radicals, comprising one or more compounds selected from the group consisting of compounds of the following formula (I) to formula (III):
(wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]) and physiologically acceptable salts thereof.

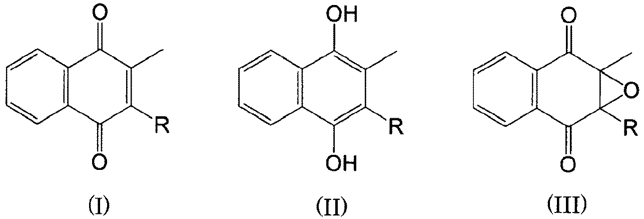

## Description

### Technical Field

The present invention relates to an agent for inhibiting ferroptosis (also pronounced as "ferro-p-tosis"), an agent for preventing or treating a disease associated with ferroptosis, an agent for scavenging (a scavenger of) lipid peroxy radicals, an agent for inhibiting lipid peroxide accumulation, and an agent for preventing or treating a disease caused by lipid peroxide accumulation and/or lipid peroxy radicals.

### Background Art

Ferroptosis is a free iron (Fe²⁺)-dependent, nonapoptotic, regulated cell death and is characterized by lipid peroxide accumulation in the cell (Non-patent Document 1). Ferroptosis has been reported to be involved in various diseases such as acute tissue injury and neurodegenerative diseases (Non-patent Document 2).

When a lipid is oxidized, or the activity of glutathione peroxidase 4 (GPX4), which is an enzyme that reduces lipid peroxides, is decreased, lipid peroxides are produced and accumulated in a cell. Then, lipid peroxy radicals are produced from the lipid peroxides produced and accumulated in the cell by Fenton reaction using Fe²⁺ as a catalyst, and lipid peroxidation is promoted by lipid peroxy radicals.

Trolox and Coenzyme Q10, which are antioxidant substances, and baicalein, which is an inhibitor of lipoxygenase involved in production of lipid peroxides, are known to have an effect of inhibiting ferroptosis. Further, a recent report has shown that a specific spiroquinoxaline derivative has an inhibitory effect on ferroptosis (Patent Document 1). Further, the present inventors have found low molecular weight compounds that have effects of specifically scavenging lipid peroxy radicals, inhibiting lipid peroxide accumulation and lipid peroxy radical production, and inhibiting ferroptosis caused by lipid peroxides and lipid peroxy radicals (Japanese Patent Application No. 2019-227405) .

### Prior Art Documents

### Patent Document

Patent Document 1: Japanese unexamined Patent Application Publication No. 2019-196390

### Non-patent Documents

Non-patent Document 1: Cell. 2012 May 25; 149(5) :1060-72.
Non-patent Document 2: Cell. 2017 Oct 5; 171(2):273-285.

### Summary of the Invention

### Object to be Solved by the Invention

An object of the present invention is to provide a low molecular weight compound having an anti-ferroptosis activity and an ability to scavenge lipid peroxy radicals which is relatively highly safe when ingested, can be orally ingested and can be manufactured at a relatively low cost.

### Means to Solve the Object

The present inventors are continuing to study assiduously to solve the above-mentioned object. During this process, compounds having an anti-ferroptosis activity were screened on the basis of a finding that substrates of cytochrome P450 and compounds inducing the enzyme have an ability to inhibit ferroptosis. As a result, the present inventors found in *in vitro* and *in vivo* analyses that the vitamin K group compounds of the present invention described later had an excellent anti-ferroptosis activity. Further, it has been confirmed that the vitamin K group compounds of the present invention have an inhibitory effect on lipid peroxide accumulation and an ability to scavenge lipid peroxy radicals. The present invention has been accomplished on the basis of these findings.

That is, the present invention provides the following:
[1] An agent for inhibiting ferroptosis comprising one or more compounds selected from the group consisting of compounds of the following formula (I): 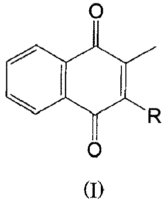 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]),
   the following formula (II): 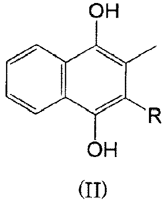 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]), and
   the following formula (III): 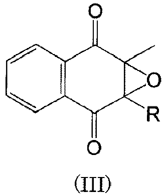 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12])
      and physiologically acceptable salts thereof (hereinafter may be referred to as "the vitamin K group compounds of the present invention").
[2] An agent for preventing or treating a disease associated with ferroptosis comprising one or more compounds selected from the group consisting of compounds of the following formula (I): 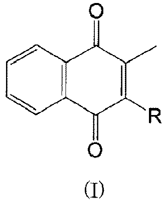 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]),
   the following formula (II): 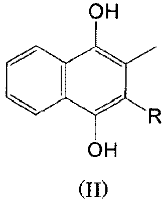 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]), and
   the following formula (III): 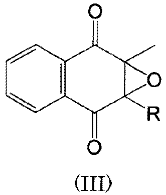 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12])
      and physiologically acceptable salts thereof.
[3] An agent for scavenging lipid peroxy radical comprising one or more compounds selected from the group consisting of compounds of the following formula (I): 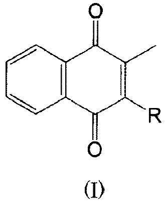 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]),
   the following formula (II): 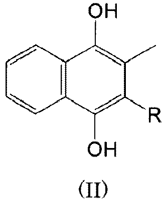 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]), and
   the following formula (III): 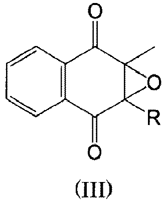 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]),
      and physiologically acceptable salts thereof.
[4] An agent for inhibiting lipid peroxide accumulation comprising one or more compounds selected from the group consisting of compounds of the following formula (I): 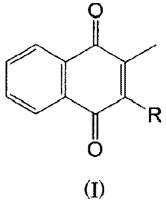 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]),
   the following formula (II): 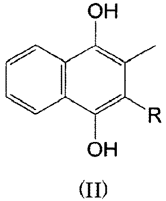 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]), and
   the following formula (III): 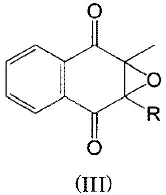 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12])
      and physiologically acceptable salts thereof.
[5] An agent for preventing or treating a disease caused by lipid peroxide accumulation and/or lipid peroxy radicals comprising one or more compounds selected from the group consisting of compounds of the following formula (I): 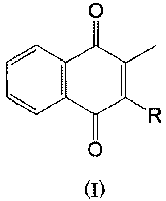 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]),
   the following formula (II): 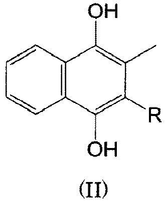 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12]), and
   the following formula (III): 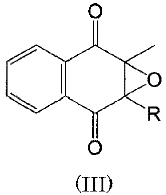 (wherein R represents H; a substituted or unsubstituted, linear or branched C₁₋₂₀ alkyl group; a substituted or unsubstituted, linear or branched C₂₋₂₀ alkenyl group; or - (CH₂CH=CH(CH₃)CH₂)ₙH [wherein n represents an integer of 1 to 12])
      and physiologically acceptable salts thereof.
[6] The agent according to the above-mentioned [2] or [5], wherein the disease is a hepatic disorder.
[7] The agent according to any one of the above-mentioned [1] to [6], which is orally ingested.
[8] The agent according to any one of the above-mentioned [1] to [7], wherein the compound of formula (I) is any one of the following (Ia) to (Ic): 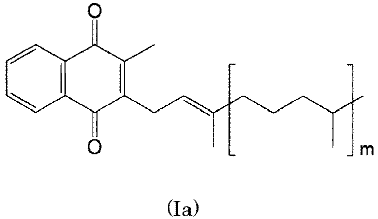 (wherein in the formula (Ia), m represents an integer of 1 to 6), 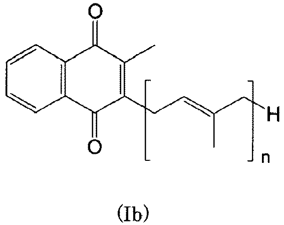 (wherein in the formula (Ib), n represents an integer of 1 to 12), and 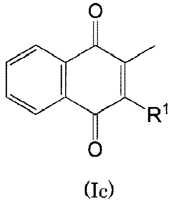 (wherein in the formula (Ic), R¹ represents H or a substituted or unsubstituted, linear or branched C₁₋₂₀ alkyl group).
[9] The agent according to any one of the above-mentioned [1] to [8], wherein the compound of formula (II) is any one of the following (IIa) to (IIc): 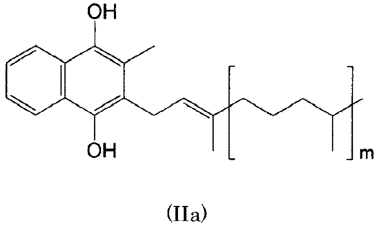 (wherein in the formula (IIa), m represents an integer of 1 to 6), 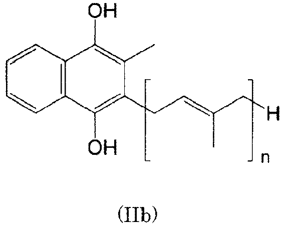 (wherein in the formula (IIb), n represents an integer of 1 to 12), and 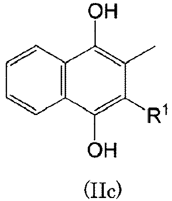 (wherein in the formula (IIc), R¹ represents H or a substituted or unsubstituted, linear or branched C₁₋₂₀ alkyl group).
[10] The agent according to any one of the above-mentioned [1] to [9], wherein the compound of formula (III) is any one of the following (IIIa) to (IIIc): 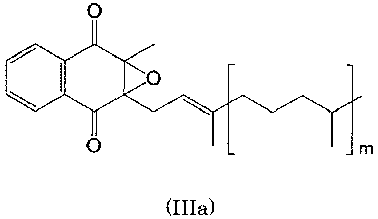 (wherein in the formula (IIIa), m represents an integer of 1 to 6), 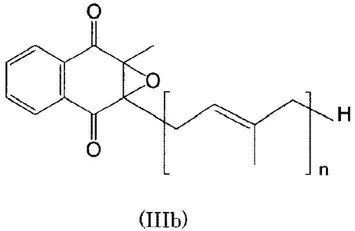 (wherein in the formula (IIIb), n represents an integer of 1 to 12), and 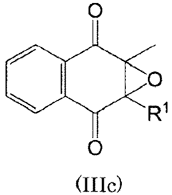 (wherein in formula (IIIc), R¹ represents H or a substituted or unsubstituted, linear or branched C₁₋₂₀ alkyl group).
[11] The agent according to any one of the above-mentioned [1] to [10], wherein the compound is any one of the following:

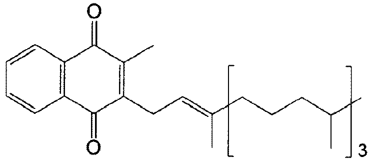

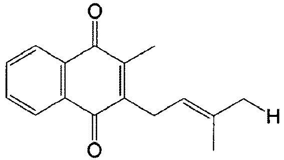

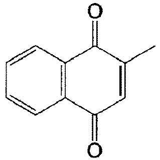

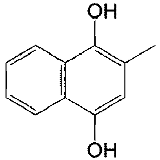

Further, examples of other embodiments of the present invention include the following:
a method for inhibiting ferroptosis, comprising a step of allowing a subject in need of inhibition of ferroptosis to ingest the vitamin K group compounds of the present invention;
the vitamin K group compounds of the present invention for use as an agent for inhibiting ferroptosis;
the vitamin K group compounds of the present invention for use in inhibition of ferroptosis; and
use of the vitamin K group compounds of the present invention for manufacturing an agent for inhibiting ferroptosis.

Further, examples of other embodiments of the present invention include the following:
a method for preventing or treating a disease associated with ferroptosis, comprising a step of allowing a subject in need of prevention or treatment of a disease associated with ferroptosis to ingest (administering) the vitamin K group compounds of the present invention;
the vitamin K group compounds of the present invention for use as an agent for preventing or treating a disease associated with ferroptosis;
the vitamin K group compounds of the present invention for use in prevention or treatment of a disease associated with ferroptosis; and
use of the vitamin K group compounds of the present invention for manufacturing an agent for preventing or treating a disease associated with ferroptosis.

Further, examples of other embodiments of the present invention include the following:
a method for scavenging lipid peroxy radicals, comprising a step of allowing a subject in need of scavenging of lipid peroxy radicals to ingest (administering) the vitamin K group compound of the present invention;
the vitamin K group compounds of the present invention for use as an agent for scavenging lipid peroxy radical;
the vitamin K group compound of the present invention for use in scavenging of lipid peroxy radicals; and
use of the vitamin K group compounds of the present invention for manufacturing an agent for scavenging lipid peroxy radical.

Further, examples of other embodiments of the present invention include the following:
a method for inhibiting lipid peroxide accumulation, comprising a step of allowing a subject in need of inhibition of lipid peroxide accumulation to ingest the vitamin K group compounds of the present invention;
the vitamin K group compounds of the present invention for use as an agent for inhibiting lipid peroxide accumulation;
the vitamin K group compound of the present invention for use in inhibition of lipid peroxide accumulation; and
use of the vitamin K group compounds of the present invention for manufacturing an agent for inhibiting lipid peroxide accumulation.

Further, examples of other embodiments of the present invention include the following:
a method for preventing or treating a disease caused by lipid peroxide accumulation and/or lipid peroxy radicals, comprising a step of allowing a subject in need of prevention or treatment of a disease caused by lipid peroxide accumulation and/or lipid peroxy radicals to ingest (administering) the vitamin K group compounds of the present invention;
the vitamin K group compounds of the present invention for use as an agent for preventing or treating a disease caused by lipid peroxide accumulation and/or lipid peroxy radicals;
the vitamin K group compounds of the present invention for use in prevention or treatment of a disease caused by lipid peroxide accumulation and/or lipid peroxy radicals; and
use of the vitamin K group compounds of the present invention for manufacturing an agent for preventing or treating a disease caused by lipid peroxide accumulation and/or lipid peroxy radicals.

### Effect of the Invention

The vitamin K group compounds of the present invention have an anti-ferroptosis activity, an inhibitory effect on lipid peroxide accumulation, and/or an ability to scavenge lipid peroxy radicals and exhibits an effect of preventing and treating a disease associated with ferroptosis and a disease caused by lipid peroxide accumulation and/or lipid peroxy radicals (for example, a hepatic disorder). Further, given that the vitamin K group compounds of the present invention are a low molecular weight compounds related to vitamin K present in the body, it is relatively highly safe when ingested, can be orally ingested, and can be manufactured at a relatively low cost. Additionally, the vitamin K group compounds of the present invention are expected to pass through the blood-brain barrier (BBB) and have an effect of preventing and treating the above-mentioned diseases in the nervous system.

### Brief Description of Drawings

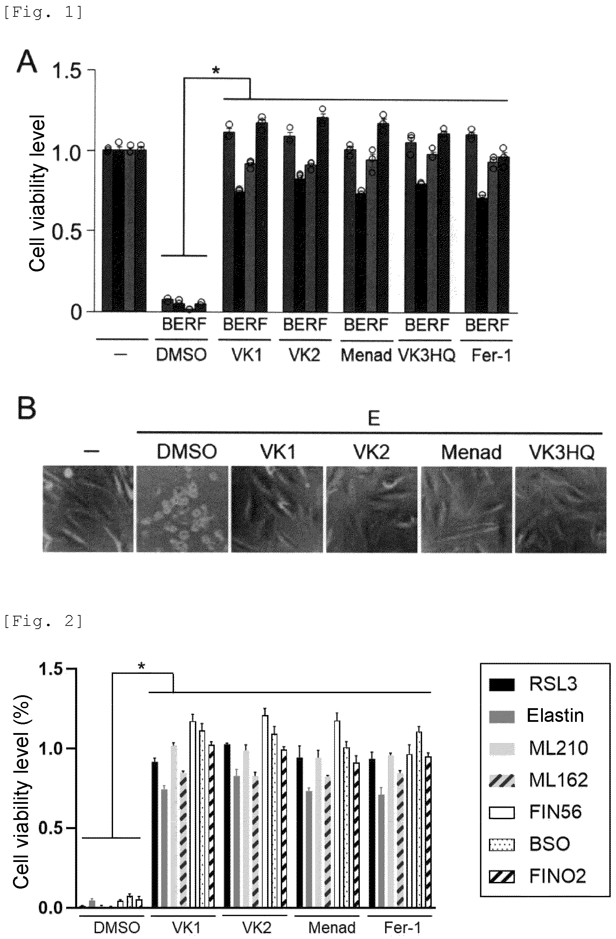

[Figure 1] Figure 1 shows the results of an analysis (n = 3) of the cell viability levels (mean ± standard error) (Figure 1A) and the phase-contrast images of cells (Figure 1B) after cells of the H9C2 cell line were cultured in the presence of each of five different test substances (vitamin K1 ["VK1" in the figure], vitamin K2 [menaquinone-4; "VK2" in the figure], menadione [vitamin K3; "Menad" in the figure], vitamin K3 hydroquinone ["VK3HQ" in the figure], or ferrostatin 1 ["Fer-1" in the figure]) and each of four different ferroptosis inducers (BSO ["B" in the figure], elastin ["E" in the figure], RSL3 ["R" in the figure], or FIN56 ["F" in the figure]). "-" in the figure shows the cell viability level when cells were cultured in the absence of those test substances and ferroptosis inducers. "DMSO" in the figure shows the cell viability level when cells were cultured in the presence of 0.1% DMSO, which is a solvent for test substances, and each of the above-mentioned ferroptosis inducers. "*" in Figure 1A indicates that there was a statistically significant difference (p < 0.05) in an ANOVA test (Dunnett's test).
[Figure 2] Figure 2 shows the results of an analysis (n = 3) of the cell viability levels (mean ± standard error) after cells of the H9C2 cell line were cultured in the presence of each of four different test substances (vitamin K1 ["VK1" in the figure], vitamin K2 [menaquinone-4; "VK2" in the figure], menadione [vitamin K3; "Menad" in the figure], or ferrostatin 1 ["Fer-1" in the figure]) and each of seven different ferroptosis inducers (RSL3, elastin, ML210, ML162, FIN56, BSO, or FINO2). "DMSO" in the figure shows the cell viability levels when cells were cultured in the presence of 0.1% DMSO, which is a solvent for test substances, and each of the above-mentioned four different ferroptosis inducers. "*" in the figure indicates that there was a statistically significant difference (p < 0.05) in an ANOVA test (Dunnett's test).

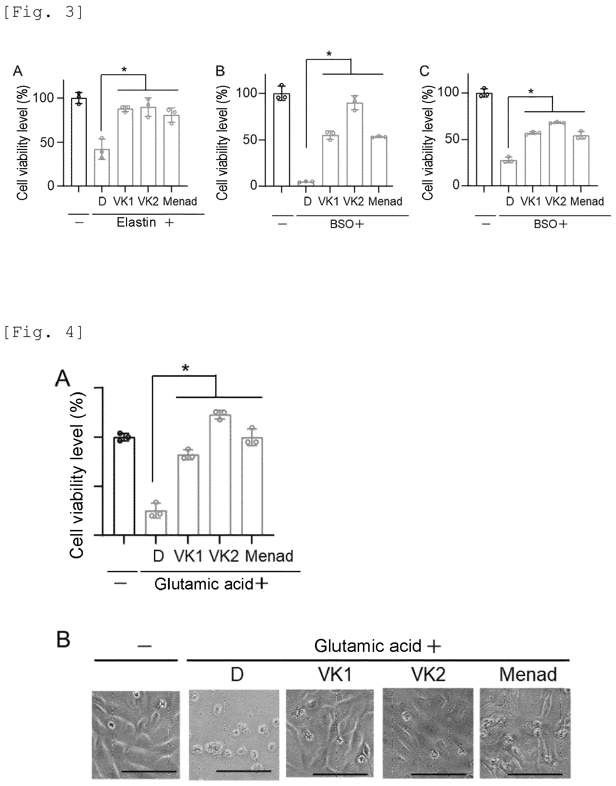

[Figure 3] Figure 3A shows the results of an analysis (n = 3) of the cell viability levels (mean ± standard error) after cells of the Panc-1 cell line were cultured in the presence of each of three different test substances (vitamin K1 ["VK1" in the figure], vitamin K2 [menaquinone-4; "VK2" in the figure], or menadione [vitamin K3; "Menad" in the figure]) and a ferroptosis inducer (elastin). Figure 3B shows the results of an analysis (n = 3) of the cell viability levels (mean ± standard error) after cells of the NRK49F cell line were cultured in the presence of each of the above-mentioned three different test substances and a ferroptosis inducer (BSO). Figure 3C shows the results of an analysis (n = 3) of the cell viability levels (mean ± standard error) after cells of the C2C12 cell line were cultured in the presence of each of the above-mentioned three different test substances and a ferroptosis inducer (BSO). "-" in the figure shows the cell viability levels when cells were cultured in the absence of those test substances and a ferroptosis inducer, and "D" in the figure shows the cell viability levels when cells were cultured in the presence of 0.1% DMSO, which is a solvent for test substances, and a ferroptosis inducer (elastin or BSO). "*" in the figure indicates that there was a statistically significant difference (p < 0.05) in an ANOVA test (Dunnett's test).
[Figure 4] Figure 4 shows the results of an analysis (n = 3) of the cell viability levels (mean ± standard error) (Figure 4A) and the phase-contrast images of cells (Figure 4B) after cells from the HT22 cell line were cultured in the presence of each of three different test substances (vitamin K1 ["VK1" in the figure], vitamin K2 [menaquinone-4; "VK2" in the figure], or menadione [vitamin K3; "Menad" in the figure]) and a ferroptosis inducer (glutamic acid). "-" in the figure shows the cell viability level when cells were cultured in the absence of those test substances and a ferroptosis inducer. "D" in the figure shows the cell viability level when cells were cultured in the presence of 0.1% DMSO, which is a solvent for test substances, and a ferroptosis inducer (glutamic acid). The scale bar in the images of Figure 4B represents 100 µm. "*" in the figure indicates that there was a statistically significant difference (p < 0.05) in an ANOVA test (Dunnett's test).

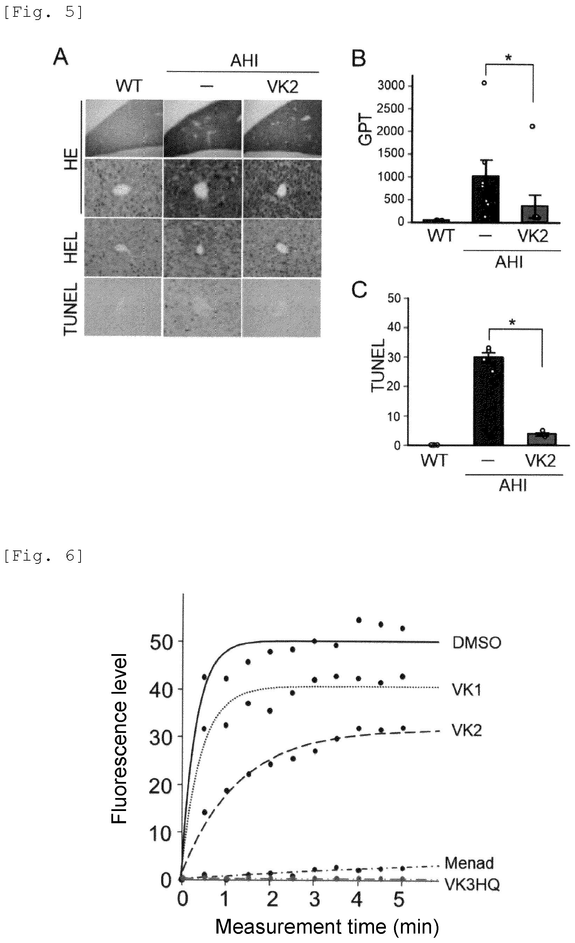

[Figure 5] Figure 5A shows the results of a hematoxylin and eosin staining method ("HE" in the figure), an immunohistological staining method using an anti-HEL antibody ("HEL" in the figure), and a TUNEL method ("TUNEL" in the figure) on liver tissue sections prepared from three different groups of mice (the wild type group ["WT" in the figure], the AHI/solvent treatment group ["AHI-" in the figure], and the AHI/VK2 treatment group ["AHI VK2" in the figure]). The HE images in the lower column are high-magnification images of the HE images in the upper column. Figure 5B shows the results of measurement of glutamate pyruvate transaminase (GPT) concentrations in plasma in the above-mentioned three groups of mice. Figure 5C shows the results of measurement of the staining levels detected by the TUNEL method in Figure 5A. "*" in Figures 5B and 5C indicates that there was a statistically significant difference (p < 0.05) in an ANOVA test (Dunnett's test).
[Figure 6] Figure 6 shows the results of an analysis of a scavenging ability of four different test substances (vitamin K1 ["VK1" in the figure], vitamin K2 [menaquinone-4; "VK2" in the figure], menadione [vitamin K3; "Menad" in the figure], and vitamin K3 hydroquinone ["VK3HQ" in the figure]) against lipid peroxy radicals by the arachidonic acid (AA)/lipoxygenase (LOX) system using an NBD-Pen fluorescence probe. "DMSO" in the figure shows the result of a similar analysis using 0.1% DMSO, which is a solvent for test substances.

### Mode of Carrying Out the Invention

The agent for inhibiting ferroptosis of the present invention is an agent comprising the vitamin K group compound of the present invention identified for the use "to inhibit ferroptosis" (hereinafter may be referred to as "the ferroptosis inhibitor of the present invention").

The agent for preventing or treating a disease associated with ferroptosis of the present invention is an agent comprising the vitamin K group compound of the present invention identified for the use "to prevent or treat a disease associated with ferroptosis" (hereinafter may be referred to as "the preventing/treating agent of the present invention 1").

The agent for scavenging lipid peroxy radical of the present invention is an agent comprising the vitamin K group compound of the present invention identified for the use "to scavenge lipid peroxy radicals produced in various organs or tissues in an organism, cultured cell lines derived from various organs or tissue in an organism, and the like" (hereinafter may be referred to as "the scavenger of the present invention"). Here, the expression "scavenging lipid peroxy radicals" means inhibiting a reaction in which lipid peroxy radicals are produced, more specifically, a reaction in which lipid peroxy radicals are produced from lipid peroxides (also referred to as "LOOH") by Fenton reaction using Fe²⁺ as a catalyst. Therefore, an "agent for scavenging lipid peroxy radical" is, in other words, an "agent for inhibiting production of lipid peroxy radicals."

The agent for inhibiting lipid peroxide accumulation of the present invention is an agent comprising the vitamin K group compound of the present invention identified for use "to inhibit accumulation of lipid peroxides produced in various organs or tissues in an organism, cultured cell lines derived from various organs or tissue in an organism, and the like" (hereinafter may be referred to as "the lipid peroxide accumulation inhibitor of the present invention").

The agent for preventing or treating a disease caused by lipid peroxides and/or lipid peroxy radicals of the present invention is an agent comprising the vitamin K group compound of the present invention identified for use "to prevent or treat a disease caused by lipid peroxides and/or lipid peroxy radicals" (hereinafter may be referred to as "the preventing/treating agent of the present invention 2").

For the ferroptosis inhibitor of the present invention, the scavenger of the present invention, and the lipid peroxide accumulation inhibitor of the present invention, the vitamin K group compound of the present invention may be used alone as a feed for non-human mammals, a food or drink, or a drug (a pharmaceutical formulation), or may be used in a form of a composition (that is, a feed composition for non-human mammals, a food or drink composition, or a pharmaceutical composition) by further mixing additives. Examples of the above-mentioned food and drink include a health food (for example, a functional food, a nutritive supplement food, a health supplement food, a nutrient fortified food, a nutrient controlling food, and a supplement) and a health function food (for example, a food for specified health use, a nutritive functional food, and a function labeled food).

For the preventing/treating agent of the present invention 1 and the preventing/treating agent of the present invention 2, the vitamin K group compound of the present invention may be used alone as a drug for non-human mammals, or a drug (a pharmaceutical formulation), or may be used in a form of a composition (that is, a pharmaceutical composition for non-human mammals, or a pharmaceutical composition for human) by further mixing additives. Of note, the ferroptosis inhibitor of the present invention, the preventing/treating agent of the present invention 1, the scavenger of the present invention, the lipid peroxide accumulation inhibitor of the present invention, and the preventing/treating agent of the present invention 2 may be hereinafter referred to as "the agent of the present invention" as a collective term.

In the present specification, "lipid peroxy radical" means a radical (that is, an atom or a molecule having an unpaired electron; also referred to as a "free radical") of a lipid peroxide and is one type of active oxygen which is highly reactive and is also expressed as "LOO•." A lipid peroxy radical is produced from LOOH by Fenton reaction using Fe²⁺ as a catalyst, and is also produced by a reaction of a lipid radical (L•) and an oxygen molecule. Further, a lipid peroxide is produced and accumulated from a lipid (LH), for example, by a peroxidation reaction by a free radical such as a lipid peroxy radical, or by reduced GPX4 activity and/or System Xc-function.

In the present specification, "ferroptosis" means cell death (more specifically, active or regulated cell death) caused by lipid peroxide accumulation, a lipid peroxy radical, a superoxide anion radical (O₂⁻•), a hydroxy radical (•OH), or the like in various organs or tissues in the body and cultured cell lines derived from various organs or tissue in the body, and is different from cell deaths other than ferroptosis (for example, apoptosis, necrosis, and autophagic cell death). Examples of those various organs or tissues include the large intestine (colon or rectum), stomach, liver, heart, brain, spinal cord, lung, esophagus, duodenum, small intestine, skin, prostate, bladder, uterus, kidney, pancreas, spleen, trachea, bronchus, gallbladder, and bile duct.

The above-mentioned "disease associated with ferroptosis" may be any disease associated with ferroptosis (that is, a disease caused by ferroptosis and/or a disease accompanied by ferroptosis) in the above-mentioned various organs or tissues. Further, the above-mentioned "disease caused by lipid peroxides and/or lipid peroxy radicals" is may be any disease caused by lipid peroxide accumulation and/or lipid peroxy radical production (more specifically, a disease characterized by ferroptosis) in various organs or tissues. Examples of these diseases include a dysfunction in the above-mentioned various organs or tissues, for example, a disorder in the eyes (that is, an eye disease), such as retinitis pigmentosa, age-related macular degeneration, glaucoma, and cataract (refer to "Cells. 2021 Aug 21; 10(8):2153. doi: 10.3390/cells10082153.," "Int J Mol Sci. 2020 Oct 1; 21(19):7279. doi: 10.3390/ijms21197279.," "Exp Eye Res. 2020 Feb; 191:107922. doi: 10.1016/j.exer.2020.107922. Epub 2020 Jan 7.," and "Free Radic Biol Med. 2021 May 1; 167:94-108. doi: 10.1016/j.freeradbiomed.2021.02.010. Epub 2021 Mar 17."); a disorder in the lungs (that is, a lung disease), such as chronic obstructive pulmonary disease and pulmonary fibrosis; a disorder in the liver (that is, a liver disease), such as fatty liver and hepatitis; a disorder in the skin (that is, a skin disease); a disorder in the kidneys (that is, a kidney disease), such as acute nephropathy; a disorder in the large intestines (that is, a colorectal disease), such as ulcerative colitis; a disorder in the heart (that is, a heart disease), such as angina pectoris, myocardial infarction, and arteriosclerosis; a disorder in the nerve (that is, a neurological disease), such as traumatic brain damage, Alzheimer's disease, Parkinson's disease, amyotrophic lateral sclerosis, Huntington's disease, ischemic brain disease, and cerebral infarction; a mitochondrial disease; and a cancer such as granuloma. Since the effect has been proved in the example described later, a preferred example among these diseases is a hepatic disorder. The damaged tissue, internal organ or lesion in the above-mentioned mitochondrial disease may be a single tissue, organ, or internal organ or may be a plurality of tissues, organs, or internal organs. Examples of the tissue, organ, or internal organ include the central nerve, skeletal muscle, heart, eye, liver, kidney, pancreas, blood, inner ear, large intestine, small intestine, and skin. Further, the disease associated with ferroptosis and the disease caused by lipid peroxides and/or lipid peroxy radicals may be an acute disease or a chronic disease, but a preferred example is an acute disease (in particular, an acute hepatic injury) because the effects are proved in the example described later. Further, since the example described later shows that the vitamin K group compounds of the present invention inhibit death of cells from the HT22 cell line caused by glutamic acid (that is, ferroptosis in nerve cells), a preferred example of the above-mentioned "disease associated with ferroptosis" and the above-mentioned "disease caused by lipid peroxides and/or lipid peroxy radicals" is a neurological disease.

In the present invention, the vitamin K group compounds of the present invention mean compounds of the following formulas (I), (II), and (III) and physiologically acceptable salts thereof.

Compound of the following formula (I):

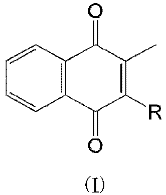

In the formula, R represents H; a substituted or unsubstituted, linear or branched C₁₋₂₀ alkyl group; a substituted or unsubstituted, linear or branched C₂₋₂₀ alkenyl group; or -(CH₂CH=CH(CH₃)CH₂)ₙH (wherein n represents an integer of 1 to 12).

The compound of the formula (I) encompasses a vitamin K compound and a derivative thereof. Examples of the vitamin K compound include vitamin K1 (also referred to as phylloquinone or phytomenadione [may be pronounced as "fi-to-menadione"]), vitamin K2 (also referred to as menaquinone), and vitamin K3 (also referred to as menadione).

Compound of the following formula (II):

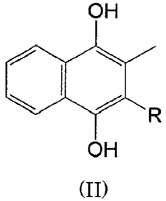

In the formula, R represents H; a substituted or unsubstituted, linear or branched C₁₋₂₀ alkyl group; a substituted or unsubstituted, linear or branched C₂₋₂₀ alkenyl group; or -(CH₂CH=CH(CH₃)CH₂)ₙH (wherein n represents an integer of 1 to 12).

The compound of formula (II) encompasses a vitamin K hydroquinone compound and a derivative thereof. Examples of the vitamin K hydroquinone compound include vitamin K1 hydroquinone, vitamin K2 hydroquinone, and vitamin K3 hydroquinone (also referred to as vitamin K4 or menadiol) . Vitamin K1 hydroquinone, vitamin K2 hydroquinone, and vitamin K3 hydroquinone are reduced forms of vitamin K1, vitamin K2, and vitamin K3, respectively.

Compound of the following formula (III):

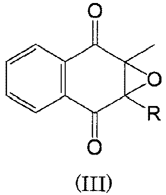

In the formula, R represents H; a substituted or unsubstituted, linear or branched C₁₋₂₀ alkyl group; a substituted or unsubstituted, linear or branched C₂₋₂₀ alkenyl group; or -(CH₂CH=CH(CH₃)CH₂)ₙH (wherein n represents an integer of 1 to 12).

The compound of formula (III) encompasses a vitamin K epoxide compound and a derivative thereof. Examples of the vitamin K epoxide compound include vitamin K1 epoxide (phylloquinone epoxide) and vitamin K3 epoxide (menadione epoxide).

In the term "substituted or unsubstituted" used in the present application, "substituted" means having one or more substituents, and "unsubstituted" means having no substituent. Examples of the substituents include a halogen atom (a fluorine atom [a fluoro group], a chlorine atom [a chloro group], a bromine atom [a bromo group], and an iodine atom [an iodo group]), an alkyl group having one to four carbon atoms, an alkoxy group having one to four carbon atoms, a hydroxy group, an amino group, a mono(C₁₋₆)alkylamino group, a di(C₁₋₆)alkylamino group, an acetyl group, a carboxyl group, a nitro group, a cyano group, a methoxy group, an ethoxy group, a trifluoromethyl group, a pentafluoroethyl group, and a phenyl group. When there are two or more substituents, each substituent is independently selected and may be identical to or different from each other. Of note, "(C₁₋₆)" means one to six carbon atoms.

The term "linear" used in the present application means a structure in which atoms other than a hydrogen atom are linked to each other linearly without being branched.

The term "branched" used in this application means a structure in which one or more arbitrary carbon atoms are bonded to two or more other carbon atoms.

In the present invention, an alkyl group has one to 20 carbon atoms. In one aspect of the present invention, an alkyl group has one to 20 carbon atoms. In one aspect of the present invention, an alkyl group has 1 to 15 carbon atoms. In one aspect of the present invention, an alkyl group has one to 10 carbon atoms. In one aspect of the present invention, an alkyl group has one to five carbon atoms.

Examples of the "alkyl group" include a methyl group, an ethyl group, a n-propyl group, an isopropyl group, a n-butyl group, an isobutyl group, a sec-butyl group, a tert-butyl group, a n-pentyl group, a 1-methylbutyl group, a 2-methylbutyl group, a 3-methylbutyl group, a 1-ethylpropyl group, a 1,1-dimethylpropyl group, a 1,2-dimethylpropyl group, a n-hexyl group, a n-decyl group, a n-pentadecyl group, and a n-eicosanyl group (n-didecyl group). The n-eicosanyl group is a linear alkyl group having 20 carbon atoms.

The term "halogen" used in the present application means a fluorine atom (F), a chlorine atom (Cl), a bromine atom (Br), and an iodine atom (I).

In the present invention, an alkenyl group has two to 20 carbon atoms. In one aspect of the present invention, an alkenyl group has two to 20 carbon atoms. In one aspect of the present invention, an alkenyl group has two to 15 carbon atoms. In one aspect of the present invention, an alkenyl group has two to 10 carbon atoms. In one aspect of the present invention, an alkenyl group has two to five carbon atoms.

Examples of the above-mentioned "alkenyl group" include an ethenyl group (vinyl group), a 1-propenyl group, a 2-propenyl group (allyl group), a 1-butenyl group, a 2-butenyl group, a 3-butenyl group, an isobutenyl group, a 1-pentenyl group, a 2-pentenyl group, a 3-pentenyl group, a 4-pentenyl group, a 1-hexenyl group, a 2-hexenyl group, a 3-hexenyl group, a 1-decenyl group, a 2-decenyl group, a 1-pentadecenyl group, a 2-pentadecenyl group, a 1-eicosenyl group, and a 2-eicosenyl group. The eicosenyl group is an alkenyl group having 20 carbon atoms.

When R represents "-(CH₂CH=CH(CH₃)CH₂)ₙH (wherein n represents an integer of 1 to 12)" in the compounds of formulas (I), (II), and (III), all compounds fall within the scope of the present invention as long as n represents any of 1, 2, 3, 4, 5, 6, 7, 8, 9, 10, 11, and 12. n represents the number of isoprene units constituting a side chain.

As one aspect of the compound of formula (I), compounds of the following formulas (Ia) to (Ic) can be exemplified:

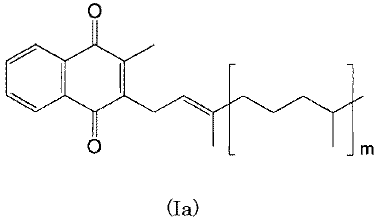

In the formula (Ia), m represents an integer of 1 to 6. When m represents 3, the compound is vitamin K1 (VK1).

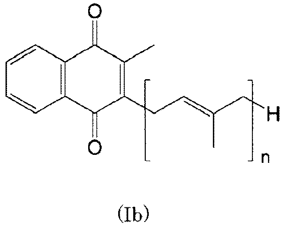

In the formula (Ib), n represents an integer of 1 to 12. The formula (Ib) represents vitamin K2. When n represents 1, the compound is menaquinone-1 (MK-1). When n represents 4, the compound is menaquinone-4 (MK-4). When n represents 7, the compound is menaquinone-7 (MK-7).

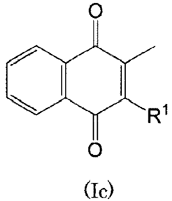

In the formula (Ic), R¹ represents H or a substituted or unsubstituted, linear or branched C₁₋₂₀ alkyl group. When R¹ represents H, the compound is menadione. In the specification and the drawings of the present application, menadione may be abbreviated as "Menad." Menadione is also referred to as vitamin K3 (VK3).

As one aspect of the compound of formula (II), compounds of the following formulas (IIa) to (IIc) can be exemplified:

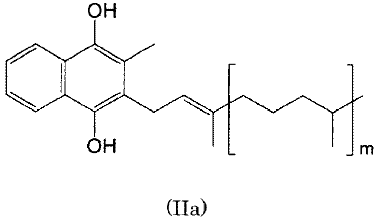

In the formula (IIa), m represents an integer of 1 to 6. When m represents 3, the compound is a hydroquinone form of vitamin K1 (VK1).

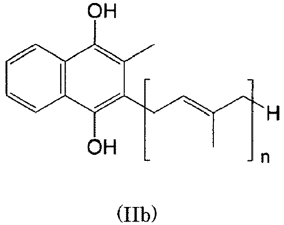

In the formula (IIb), n represents an integer of 1 to 12. When n represents 1, the compound is a hydroquinone form of MK-1, which is one type of vitamin K2 (VK2).

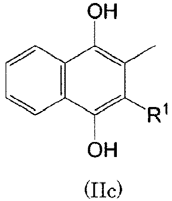

In the formula (IIc), R¹ represents H or a substituted or unsubstituted, linear or branched C₁₋₂₀ alkyl group. When R¹ represents H, the compound is a hydroquinone form of vitamin K3 (VK3).

As one aspect of the compound of formula (III), compounds of the following formulas (IIIa) to (IIIc) can be exemplified:

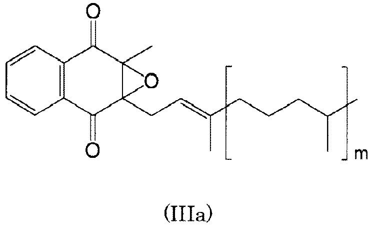

In the formula (IIIa), m represents an integer of 1 to 6. When m represents 3, the compound is an epoxide of vitamin K1 (VK1).

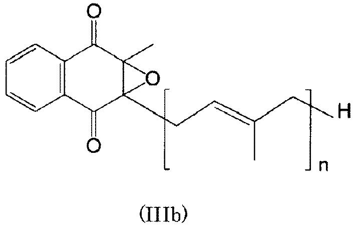

In the formula (IIIb), n represents an integer of 1 to 12. When n represents 1, the compound is an epoxide of vitamin K2 (VK2).

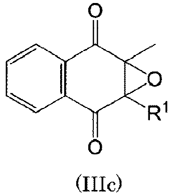

In the formula (IIIc), R¹ represents H or a substituted or unsubstituted, linear or branched C₁₋₂₀ alkyl group. When R¹ represents H, the compound is an epoxide of vitamin K3 (VK3).

The compounds of formulas (I), (II), and (III) of the present invention can be purchased as commercially available products or synthesized by suitably performing known reactions in combination. Raw materials can be purchased as commercially available products or synthesized by adding a common protective group or the like to a commercially purchased product. Further, synthesis, purification, and the like by protection and deprotection commonly used in organic chemistry can be applied to any process.

In the present specification, "physiologically acceptable salt" means a salt which, within a reasonable range of medical, pharmaceutical, or biological considerations, is commensurate with appropriate benefit-risk ratio without causing excessive toxicity, irritation, allergic response, or other problems or complications for use in contact with a mammal tissues. Examples of the physiologically acceptable salt include an ammonium salt; an alkali metal salt, such as a sodium salt, a lithium salt, and a potassium salt; an alkaline earth metal salt, such as an aluminium salt, a calcium salt, and a magnesium salt; a salt with an organic base, such as a dicyclohexylamine salt and an N-methyl-D-glucamine; a salt with an amino acid such as arginine, lysine, or ornithine; and a salt produced by a group containing basic nitrogen.

Among the vitamin K group compounds of the present invention, various isomers (for example, an optical isomer, a regioisomer, a tautomer), solvates such as a hydrate, crystal polymorphisms, and prodrugs such as an ester also fall within the scope of the present invention.

Further, the present invention also includes a compound in which one or more atoms constituting the compound of formulas (I), (II), or (III) of the present invention is an isotope, and a physiologically acceptable salt thereof. Examples of the isotope contained in the compound of the present invention include an isotope of hydrogen, carbon, nitrogen, oxygen, phosphorus, bromine, and chlorin, for example, ²H, ³H, ¹¹C, ¹³C, ¹⁴C, ¹³N, ¹⁵N, ¹⁵O, ¹⁷O, ¹⁸O, ⁷⁵Br, ⁷⁶Br, ⁷⁷Br, ⁸²Br, and ³⁷Cl.

As the above-mentioned compounds of formulas (I), (II), and (III), specifically, the compounds listed in the following Table 1 can be exemplified.

**[Table 1]**

| Name of compound | | Structural formula |
|---|---|---|
| Vitamin K1 | Phylloquinone | 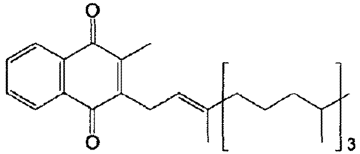 |
| Vitamin K2 | Menaquinone-1 (MK-1) | 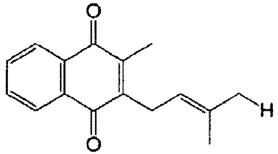 |
| | Menaquinone-4 (MK-4) | 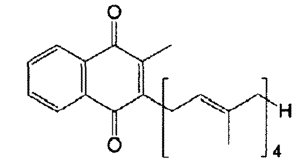 |
| | Menaquinone-7 (MK-7) | 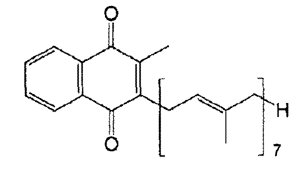 |
| Vitamin K3 | Menadione | 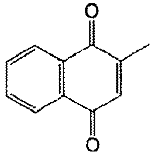 |
| Vitamin K3 hydroquinone | Menadiol | 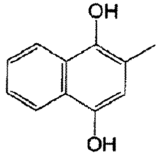 |
| Phylloquinone epoxide | | 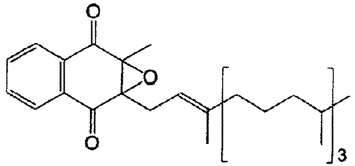 |
| Vitamin K2 (MK-1) epoxide | | 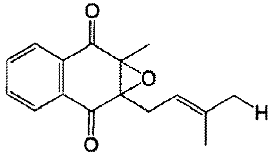 |
| Vitamin K3 epoxide | | 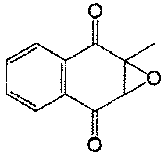 |

The vitamin K group compounds of the present invention have one or more (preferably, three) selected from an anti-ferroptosis activity; an ability to scavenge lipid peroxy radicals (that is, a scavenging ability against lipid peroxy radicals); and an inhibitory effect on lipid peroxide accumulation.

Examples of a subject to whom the agent of the present invention is allowed to ingest (administered) are not particularly limited. When the agent of the present invention is the ferroptosis inhibitor of the present invention, the subject is usually a mammal in need of inhibition of ferroptosis (for example, a mammal undergoing organ transplantation and requiring inhibition of ferroptosis that occurs in a donor organ during the organ transplantation; a mammal with a high risk of developing a disease associated with ferroptosis; a mammal suffering from such a disease). When the agent of the present invention is the scavenger of the present invention, the subject is usually a mammal in need of scavenging of lipid peroxy radicals (for example, a mammal undergoing organ transplantation and requiring scavenging of lipid peroxy radicals that are produced in a donor organ during the organ transplantation; a mammal with a high risk of developing a disease caused by lipid peroxy radicals; a mammal suffering from such a disease). When the agent of the present invention is the lipid peroxide accumulation inhibitor of the present invention, the subject is usually a mammal in need of inhibition of lipid peroxide accumulation (for example, a mammal undergoing organ transplantation and requiring inhibition of lipid peroxide accumulation that occurs in a donor organ during the organ transplantation; a mammal with a high risk of developing a disease caused by lipid peroxides, a mammal suffering from such a disease). When the agent of the present invention is the preventing/treating agent of the present invention 1, the subject is usually a mammal in need of prevention or treatment of a disease associated with ferroptosis (for example, a mammal with a high risk of developing a disease associated with ferroptosis; a mammal suffering from such a disease). When the agent of the present invention is the preventing/treating agent of the present invention 2, the subject is usually a mammal in need of prevention or treatment of a disease caused by lipid peroxides and/or lipid peroxy radicals (for example, a mammal with a high risk of developing a disease caused by lipid peroxides and/or lipid peroxy radicals; a mammal suffering from such a disease). That is, the ferroptosis inhibitor of the present invention, the scavenger of the present invention, and the lipid peroxide accumulation inhibitor of the present invention are useful for protecting a donor organ during organ transplantation.

In the present specification, examples of the mammal include a human and a non-human mammal (for example, a monkey, a mouse, a rat, a dog, a cat, a farm animal [for example, a rabbit, a pig, a horse, a cow, a sheep, a goat, a deer]).

Examples of a method for allowing a subject to ingest (administering) the agent of the present invention include a method of allowing to orally ingest (oral ingestion) it in a form of a powder, a granule, a tablet, a capsule, a syrup, a suspension, or the like (oral administration); a method of injecting it (for example, subcutaneous injection, intravenous injection, intramuscular injection, local administration) in a form (dosage form) of a solution, an emulsion, a suspension, or the like (parenteral administration); a method of intranasally administering it in a form of a spray (parenteral administration); and a method of administering it to the eyes in a form of instillation (eye drops) (instillation administration). When the preventing/treating agent of the present invention 1 and the preventing/treating agent of the present invention 2 are used for prevention or treatment of an eye disease, eye drops and a local injection to the eyes are preferred as a form of the preventing/treating agent of the present invention 1. Oral ingestion can be exemplified as a preferred method of allowing to ingest the agent of the present invention in consideration of convenience and the effect thereof proved in the example described later.

The dose of the vitamin K group compounds of the present invention is suitably determined depending on the age, body weight, sex, symptoms, susceptibility to a drug, species, and the like of a subject (mammal) to whom the compound is allowed to ingest (administered). For example, the dose range is 0.1 µg to 200 mg/kg (body weight)/day when converted to the concentration of the vitamin K group compound of the present invention. In the example described later, the vitamin K2 dose of 40 mg/kg (body weight)/day is specifically shown in an experiment using a mouse model. This dose is 3.25 mg/kg (body weight)/day when converted to the dose administered to humans on the basis of 12.3, which is the human equivalent dose (HED) in mice (refer to "Guidance for Industry Estimating the Maximum Safe Starting Dose in Initial Clinical Trials for Therapeutics in Adult Healthy Volunteers"). Therefore, the dose of the vitamin K group compound of the present invention is preferably 1.0 pg/kg (body weight)/day to 100 mg/kg (body weight)/day, more preferably 10 pg/kg (body weight)/day to 60 mg/kg (body weight)/day, further preferably 30 pg/kg (body weight)/day to 30 mg/kg (body weight)/day, further more preferably 60 pg/kg (body weight)/day to 10 mg/kg (body weight)/day, most preferably 100 pg/kg (body weight)/day to 5.0 mg/kg (body weight)/day. Of note, the vitamin K group compound of the present invention may be ingested as a single dose, or the dose may be divided into multiple doses (for example, two to four doses) per day.

In the present specification, examples of an additive include physiologically acceptable, usual ingredients for formulation, such as a carrier, a binder, a stabilizer, an excipient, a diluent, a pH buffer, a disintegrating agent, an isotonic agent, an additive, a coating agent, a solubilizer, a lubricant, a gliding agent, a dissolving aid, a lubricating agent, a flavor, a sweetener, a solvent, a gelling agent, and a nutrient. Specific examples of the ingredient for formulation include water, physiological saline, animal fat and oil, vegetable oil, lactose, starch, gelatin, crystalline cellulose, gum, talc, magnesium stearate, hydroxypropyl cellulose, polyalkylene glycol, polyvinyl alcohol, and glycerin.

The agent of the present invention may contain an anti-ferroptosis component, a component inhibiting lipid peroxide accumulation, and/or a component scavenging lipid peroxy radicals in addition to the vitamin K group compound of the present invention, which is an active ingredient, but preferably does not contain an anti-ferroptosis component, a component inhibiting lipid peroxide accumulation, and/or a component scavenging lipid peroxy radicals (for example, a compound such as a protein, an amino acid, a DNA, an RNA, and a polymer; a plant-derived extract) other than the vitamin K group compounds of the present invention, because the vitamin K group compounds of the present invention alone can exhibit an anti-ferroptosis effect, an inhibitory effect on lipid peroxide accumulation, and an effect of scavenging lipid peroxy radicals.

The present invention will be described more specifically with reference to the example, but the technical scope of the present invention is in no way limited to this example.

### Example

### 1. Materials and methods

### [Cell lines]

Three different cell lines (the H9C2 cell line, which is a rat cardiac myoblast cell line, the NRK49F cell line, which is a rat kidney fibroblast, and the C2C12 cell line, which is a mouse myoblast cell line) were obtained from the American Type Culture Collection (ATCC); the Panc-1 cell line, which is a human pancreatic cancer cell line, was obtained from the Cell Resource Center for Biomedical Research, Institute of Development, Aging and Cancer, Tohoku University/Cell Bank; and the HT22 cell line, which is a mouse hippocampus nerve cell line, was purchased from Merck Millipore Co. All the cell lines were subcultured in a Dulbecco's Modified Eagle Medium (DMEM) culture solution containing 100-fold diluted 1% penicillin-streptomycin (26253-84; manufactured by Nacalai Tesque, Inc.) (hereinafter simply referred to as "DMEM culture solution") to which 10% fetal bovine serum (FBS) and 4.5 g glucose/L were added (hereinafter referred to as "DMEM [high glucose] culture solution") under conditions of 5% CO₂/20% O₂ and 37°C.

### [Various ferroptosis induction treatments]

The experiment was performed in accordance with the following procedures [1] to [3]:
[1] 4000 cells of five different cell lines (the H9C2 cell line, the Panc-1 cell line, the NRK49F cell line, the C2C12 cell line, and the HT22 cell line) were seeded on a 96-well plate and cultured overnight in a DMEM culture solution containing 1.0 g glucose/L (low glucose) and 1% FBS (hereinafter referred to as "DMEM [low glucose low serum] culture solution").
[2] After the procedure of the above-mentioned [1], ferroptosis was induced in cells from the above-mentioned five different cell lines in the presence of each of five different vitamin K group compounds of the present invention (vitamin K1, vitamin K2 [menaquinone-4], vitamin K3, or vitamin K3 hydroquinone) (see Table 1) or a known ferroptosis inhibitor (ferrostatin 1). Specifically, cells from the H9C2 cell line were cultured in the presence of each of six different test substances (10 µM vitamin K1 [manufactured by FUJIFILM Wako Pure Chemical Corporation], 10 µM menaquinone-4 [manufactured by Sigma-Aldrich], 3 µM or 1 µM menadione [manufactured by Sigma-Aldrich], 1 µM vitamin K3 hydroquinone [manufactured by Toronto Research Chemicals], or 0.3 µM ferrostatin 1 [manufactured by Cayman]) at various concentrations and each of eight different ferroptosis inducers (100 µM BSO [manufactured by Sigma-Aldrich], 1.5 µM or 2 µM elastin [manufactured by Cayman], 50 nM RSL3 [manufactured by Selleck Chemicals], 0.3 µM ML210 [manufactured by Cayman], 0.3 µM ML162 [manufactured by Cayman], 0.5 µM FIN56 [manufactured by Cayman], 5 µM FINO2 [manufactured by Cayman], or 4 mM glutamic acid [manufactured by Sigma-Aldrich]) for 60 hours for BSO treatment, for 48 hours for elastin treatment, RSL3 treatment, and FIN56 treatment, and for 24 hours for ML210 treatment, ML162 treatment, FINO2 treatment, and glutamic acid treatment.
[3] Cell viability levels were measured by WST-8 assay using Cell Count Reagent SF. The cell viability level was calculated as a relative value of the cell survival level when the cell viability level when cultured in the absence of a test substance and a ferroptosis inducer was set to 1.

### [In vivo analysis using acute hepatic injury (AHI) model mice]

An AHI model mouse was prepared by administering lipopolysaccharide (LPS) and D-galactosamine (D-GalN). Specifically, eight to nine week-old wild-type male mice (C57BL/6N; manufactured by CLEA) were divided into three groups (the wild type group [n = 4], the AHI/solvent treatment group [n = 7], and the AHI/VK2 treatment group [n = 8]). At 12 hours and two hours before administration of LPS and D-GalN, 0.5% methylcellulose solution (solvent) was orally administered at a dose of 20 mg/kg (body weight) to the AHI/solvent treatment group, and a methylcellulose solution containing vitamin K2 (menaquinone-4) was orally administered at a dose of 20 mg (vitamin K2)/kg (body weight) to the AHI/VK2 treatment group. For LPS and D-GalN administration, PBS containing LPS and D-GalN was injected into the peritoneal cavity at a dose of 5 µg (LPS)/kg (body weight) and 500 mg (D-GalN)/kg (body weight) to the two AHI groups (that is, the AHI/solvent treatment group and the AHI/VK2 treatment group) (refer to "Sci Rep 7: 1884, 2017"). The mice were sacrificed at six hours after injection, and blood and liver tissue were collected. The plasma GPT levels were measured for the collected blood using Fuji Dri-chem Slide GPT/ALT-P III (manufactured by Fujifilm Medical) . Further, the collected liver tissue was fixed in 10% neutral buffered formalin and embedded in paraffin. The paraffin-embedded liver tissue was sliced to prepare liver tissue sections and subjected to hematoxylin and eosin (HE) staining. Further, deparaffinization treatment was performed by heating the liver tissue sections in a 0.01 M citric acid buffer solution (pH 6.0) at 120°C for five minutes to prepare deparaffinized liver tissue sections. Immunohistological staining of the deparaffinized liver tissue sections using an anti-HEL antibody (50-fold diluted; clone 5F12; manufactured by JaICA) was performed in accordance with a usual method using a streptavidin-biotin-alkaline phosphatase kit (Histofine SAB-AP[M] kit; manufactured by Nichirei Corporation) and Vector Red Substrate kit (manufactured by Vector Laboratories). Further, dead cells were detected by the TUNEL method using Apop Tag Peroxidase In Situ Apoptosis Detection Kit (manufactured by Merck Millipore).

### [Scavenging ability against lipid peroxy radicals]

The scavenging ability against lipid peroxy radicals (LOO•) was analyzed by the AA/LOX system using an NBD-Pen fluorescence probe in accordance with the method in "Nat Chem Biol 12: 608-613, 2016." Specifically, a mixture solution of 0.1% DMSO or 100 µM each of four different test substances (vitamin K1 [manufactured by FUJIFILM Wako Pure Chemical Corporation], vitamin K2 [menaquinone-4; manufactured by Sigma-Aldrich], menadione [manufactured by Sigma-Aldrich], or vitamin K3 hydroquinone [manufactured by Toronto Research Chemicals]) and PBS (pH 7.4) containing 500 µM arachidonic acid (manufactured by Sigma-Aldrich) and 1 mM dithiothreitol (DTT) was prepared and added to a 96-well plate, then 5 µM NBD-Pen fluorescence probe and 10 µg/mL LOX solution (manufactured by Sigma-Aldrich) were added, and the fluorescence level (excitation light, 470 nm; fluorescence wavelength, 530 nm) was measured using a microplate reader (Spectra Max M2e; manufactured by Molecular Devices) at 37°C.

### 2. Results

### [Various ferroptosis induction treatments]

It has been reported that L-buthionine sulfoximine (BSO) induces ferroptosis by inhibiting γ-glutamylcysteine synthetase, which is a rate limiting enzyme of glutathione synthesis (refer to "Cell, 156: 317-331, 2014" and "Biochem Biophys Res Commun, 499: 44-51, 2018"). Further, elastin and RSL3 produce lipid peroxides and induce ferroptosis by inhibiting System Xc- and GPX4, respectively (refer to "Cell Death Differ, 23: 369-379, 2016"). Further, it has been reported that a low molecular weight compound FIN56 induces ferroptosis by degrading GPX4, which inhibits lipid peroxide accumulation (refer to "Nature chemical Biology, 12: 497-503, 2016"). Further, it has been reported that ML210 is a prodrug type molecule which is activated after taken up into viable cells and induces ferroptosis by inhibiting GPX4 (refer to "Nature, 569: 270-274, 2019"). Further, it has been reported that ML162 is a substance which inhibits GPX4 more selectively and potently than RSL3 and induces ferroptosis (refer to "Bioorg Med Chem Lett, 22: 1822-1826, 2012"). Further, it has been reported that FINO2 oxidizes intracellular iron and induces ferroptosis by inhibiting GPX4 (refer to "Nat Chem Biol, 14: 507-515, 2018"). Further, it has been reported that a high concentration of extracellular glutamic acid inhibits System Xc- and induces ferroptosis (refer to "Cell, 149: 1060-1072, 2012"). Accordingly, these eight different ferroptosis inducers (BSO, elastin, RSL3, FIN56, ML210, ML162, FINO2, and glutamic acid) were used to examine whether the vitamin K group compounds of the present invention had an anti-ferroptosis activity.

When cells of the H9C2 cell line were cultured in the presence of each of four different ferroptosis inducers (100 µM BSO, 2 µM elastin, 50 nM RSL3, or 0.5 µM FIN56), the cell viability level was considerably decreased as compared with when cells were cultured in the absence of a ferroptosis inducer (see "DMSO" and "-" in Figure 1), whereas this decrease in the cell viability level was significantly suppressed when cells from the H9C2 cell line were cultured in the presence of each of four different vitamin K group compounds of the present invention (10 µM vitamin K1, 10 µM vitamin K2 [menaquinone-4], 1 µM menadione [vitamin K3], or 1 µM vitamin K3 hydroquinone) or a known ferroptosis inhibitor (0.3 µM ferrostatin 1) together with the above-mentioned four different ferroptosis inducers (see "VK1", "VK2", "Menad", "VK3HQ", and "Fer-1" in Figure 1).

Further, when cells of the H9C2 cell line were cultured in the presence of each of seven different ferroptosis inducers (50 nM RSL3, 2 µM elastin, 0.3 µM ML210, 0.3 µM ML162, 0.5 µM FIN56, 100 µM BSO, or 5 µM FINO2) and three different vitamin K group compounds of the present invention (10 µM vitamin K1, 10 µM vitamin K2 [menaquinone-4], or 3 µM menadione [vitamin K3]) or a known ferroptosis inhibitor (0.3 µM ferrostatin 1), the cell viability level was shown to be significantly increased as compared with the case where cells were cultured in the presence of the above-mentioned seven different ferroptosis inducers (see Figure 2).

Further, when cells of the Panc-1 cell line were cultured in the presence of a ferroptosis inducer (5 µM elastin), the cell viability level was considerably decreased as compared with when cells were cultured in the absence of this ferroptosis inducer (see "D" and "-" in Figure 3A), whereas this decrease in the cell viability level was significantly suppressed when cells from the Panc-1 cell line were cultured in the presence of three different vitamin K group compounds of the present invention (10 µM vitamin K1, 10 µM vitamin K2 [menaquinone-4], or 3 µM menadione [vitamin K3]) together with a ferroptosis inducer (5 µM elastin) (see Figure 3A).

Further, when cells of the NRK49F cell line and the C2C12 cell line were cultured in the presence of a ferroptosis inducer (500 µM BSO), the cell viability level was considerably decreased as compared with when cells were cultured in the absence of this ferroptosis inducer (see "D" and "-" in Figures 3B and 3C), whereas this decrease in the cell viability level was significantly suppressed when cells from the NRK49F cell line and the C2C12 cell line were cultured in the presence of three different vitamin K group compounds of the present invention (10 µM vitamin K1, 10 µM vitamin K2 [menaquinone-4], or 3 µM menadione [vitamin K3]) together with a ferroptosis inducer (500 µM BSO) (see Figures 3B and 3C).

Further, when cells of the HT22 cell line were cultured in the presence of a ferroptosis inducer (4 mM glutamic acid), the cell viability level was considerably decreased as compared with when cells were cultured in the absence of the ferroptosis inducer (see "D" and "-" in Figure 4), whereas this decrease in the cell viability level was significantly suppressed when cells from the HT22 cell line were cultured in the presence of three different vitamin K group compounds of the present invention (10 µM vitamin K1, 10 µM vitamin K2 [menaquinone-4], or 3 µM menadione [vitamin K3]) together with a ferroptosis inducer (4 mM glutamic acid) (see Figure 4).

These results indicate that the vitamin K group compounds of the present invention have an anti-ferroptosis activity as with known ferroptosis inhibitors.

### [In vivo analysis using AHI model mice]

Further, an analysis was performed using AHI model mice to confirm *in vivo* that the vitamin K group compounds of the present invention had an anti-ferroptosis activity. First, in mice of the AHI/solvent treatment group (that is, the AHI model mice), HEL, which is a lipid peroxide marker, was detected, severe hemorrhage was confirmed, the proportion of dead cells increased (see Figures 5A and 5C), and the plasma GPT levels, which is a marker of decreased liver function, were shown to increase significantly as compared with the levels in mice of the wild type group (that is, wild type mice) (see Figure 5B).

These results indicate that lipid peroxides were accumulated and lipid peroxy radicals were produced by LPS and D-GalN in the liver tissue of the AHI model mice, resulting in cell death due to ferroptosis and development of a hepatic disorder.

However, severe hemorrhage in the liver tissue observed in the mice of the AHI/solvent treatment group was relieved in the liver tissue from the mice of the AHI/VK2 treatment group (that is, the AHI model mice to which vitamin K2 [menaquinone-4] was administered), the proportion of dead cells was significantly decreased as compared with liver tissue from the mice of the AHI/solvent treatment group, and the HEL detection level was substantially decreased as compared with the HEL detection level in the liver tissue from the mice of the AHI/solvent treatment group (see Figures 5A and 5C). Further, the GPT level in the mice of the AHI/VK2 treatment group was significantly decreased as compared with the GPT level in the mice of the AHI/solvent treatment group (see Figure 5B).

These results indicate that when the vitamin K group compounds of the present invention, such as vitamin K2, were administered to the AHI model mice, lipid peroxide accumulation and lipid peroxy radical production in the liver tissue were suppressed by the effect of vitamin K2, resulting in suppression of cell death and hepatic disorder due to ferroptosis.

### [Scavenging ability against lipid peroxy radicals]

The relationship between the anti-ferroptosis effect and the scavenging ability against lipid peroxy radicals of the vitamin K group compounds of the present invention was analyzed by the AA/LOX system using an NBD-Pen fluorescence probe. In the AA/LOX system using an NBD-Pen fluorescence probe, an oxidation reaction of AA by LOX produces a lipid peroxide (LOOH), a lipid peroxy radical (LOO•) is produced from the produced LOOH by Fenton reaction using an iron ion (Fe²⁺) as a catalyst, and a scavenging ability against lipid peroxy radicals is detected using the level of NBD-Pen-derived fluorescence produced by a reaction of the produced lipid peroxy radical with NBD-Pen as an indicator.

It was shown that the fluorescence signal level was decreased when the NBD-Pen fluorescence probe was placed in the presence of each of four different test substances (vitamin K1, vitamin K2 [menaquinone-4], menadione [vitamin K3], or vitamin K3 hydroquinone) in addition to AA and LOX, as compared with when the probe was placed in the absence of these test substances (see Figure 6).

This result indicates that the vitamin K group compounds of the present invention have a scavenging ability against lipid peroxy radicals and have an anti-ferroptosis activity due to this effect.

### Industrial Applicability

The present invention contributes to prevention or treatment of a disease associated with ferroptosis or a disease caused by lipid peroxide accumulation and/or lipid peroxy radicals.

## Claims

1. An agent for inhibiting ferroptosis, comprising one or more compounds selected from the group consisting of compounds of the following formula (I): 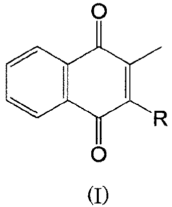 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]),
the following formula (II): 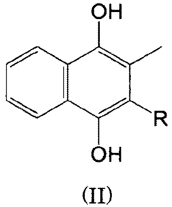 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]), and
the following formula (III) : 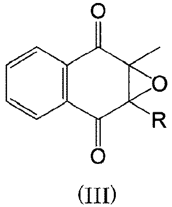 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12])
and physiologically acceptable salts thereof.

2. An agent for preventing or treating a disease associated with ferroptosis, comprising one or more compounds selected from the group consisting of compounds of the following formula (I): 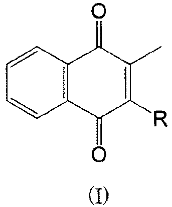 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]),
the following formula (II): 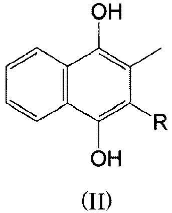 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]), and
the following formula (III) : 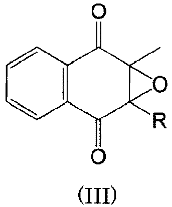 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12])
and physiologically acceptable salts thereof.

3. An agent for scavenging lipid peroxy radical, comprising one or more compounds selected from the group consisting of compounds of the following formula (I): 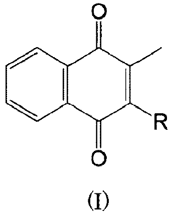 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]),
the following formula (II): 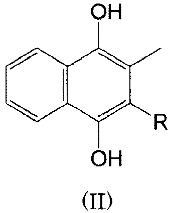 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]), and
the following formula (III) : 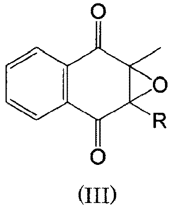 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]),
and physiologically acceptable salts thereof.

4. An agent for inhibiting lipid peroxide accumulation, comprising one or more compounds selected from the group consisting of compounds of the following formula (I): 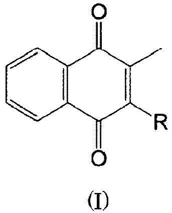 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]),
the following formula (II): 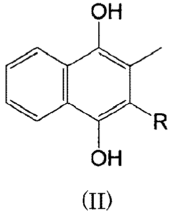 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]), and
the following formula (III) : 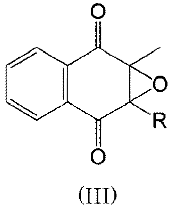 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12])
and physiologically acceptable salts thereof.

5. An agent for preventing or treating a disease caused by lipid peroxide accumulation and/or lipid peroxy radicals, comprising one or more compounds selected from the group consisting of compounds of the following formula (I): 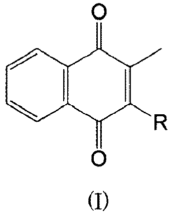 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]),
the following formula (II): 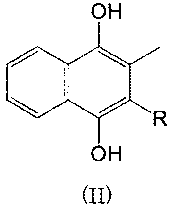 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12]), and
the following formula (III) : 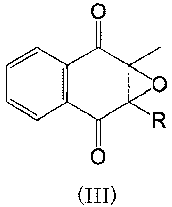 (wherein R represents H; a substituted or unsubstituted, linear or branched C₁₋₂₀ alkyl group; a substituted or unsubstituted, linear or branched C₂₋₂₀ alkenyl group; or-(CH₂CH=CH(CH₃)CH₂)ₙH [wherein n represents an integer of 1 to 12])
and physiologically acceptable salts thereof.

6. The agent according to claim 2 or 5, wherein the disease is a hepatic disorder.

7. The agent according to any one of claims 1 to 6, wherein the agent is orally ingested.

8. The agent according to any one of claims 1 to 7, wherein the compound of formula (I) is any one of the following (Ia) to (Ic) : 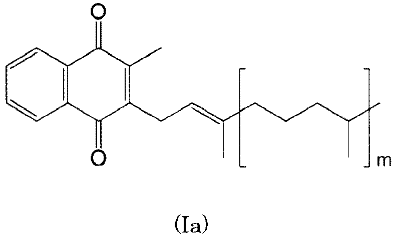 (wherein in the formula (Ia), m represents an integer of 1 to 6), 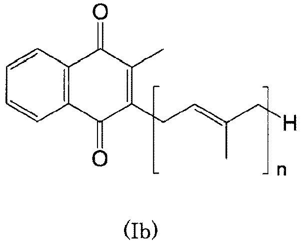 (wherein in the formula (Ib), n represents an integer of 1 to 12), and 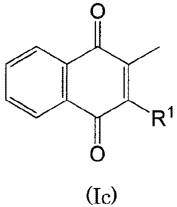 (wherein in the formula (Ic), R¹ represents H or a substituted or unsubstituted, linear or branched C₁₋₂₀ alkyl group).

9. The agent according to any one of claims 1 to 8, wherein the compound of formula (II) is any one of the following (IIa) to (IIc): 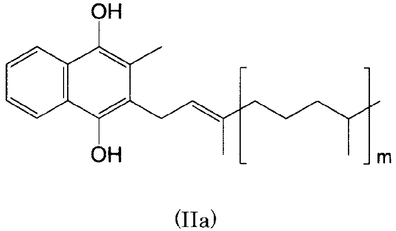 (wherein in the formula (IIa), m represents an integer of 1 to 6), 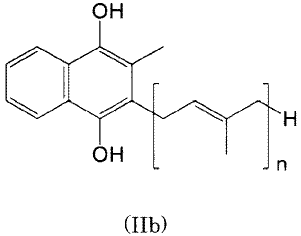 (wherein in the formula (IIb), n represents an integer of 1 to 12), and 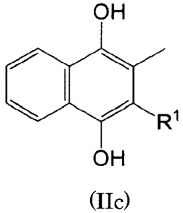 (wherein in the formula (IIc), R¹ represents H or a substituted or unsubstituted, linear or branched C₁₋₂₀ alkyl group).

10. The agent according to any one of claims 1 to 9, wherein the compound of formula (III) is any one of the following (IIIa) to (IIIc): 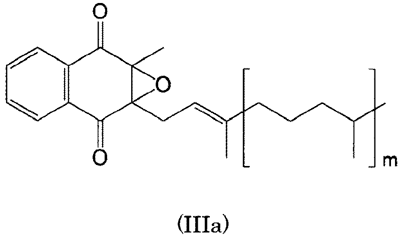 (wherein in the formula (IIIa), m represents an integer of 1 to 6), 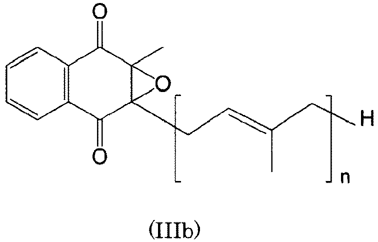 (wherein in the formula (IIIb), n represents an integer of 1 to 12), and 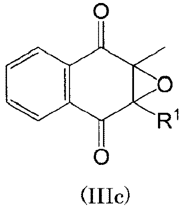 (wherein in the formula (IIIc), R¹ represents H or a substituted or unsubstituted, linear or branched C₁₋₂₀ alkyl group).

11. The agent according to any one of claims 1 to 10, wherein the compound is any one of the following:
